# EUROPEAN PATENT SPECIFICATION

(11) **EP 2 413 787 B1**
(45) Date of publication and mention of the grant of the patent: **26.07.2017**
(21) Application number: 10712933.0
(22) Date of filing: 30.03.2010
(51) Int. Cl.: A61B 5/0215, A61M 25/09

(54) **SENSOR GUIDE WIRE**
SENSOR-FÜHRUNGSDRAHT
FIL GUIDE DE CAPTEUR

(30) Priority: 31.03.2009 US 202743 P
(43) Date of publication of application: 08.02.2012
(73) Proprietor: St. Jude Medical Coordination Center BVBA, 1930 Zaventem (BE)
(72) Inventor: HILMERSSON, Mats, 168 47 Bromma (SE)
(74) Representative: Brann AB
(86) International application number: PCT/EP2010/054242
(87) International publication number: WO 2011/120565

(56) References cited:
- US-A- 5 873 835
- US-A1- 2003 028 128

## Description

### Field of the invention

The present invention relates to a sensor guide wire for intravascular measurements of physiological variables in a living body, according to the preamble of the independent claim, and in particular to a sensor guide wire for intravascular pressure measurements having a divided core wire.

### Background of the invention

In many medical procedures, various physiological conditions present within a body cavity need to be monitored. These physiological conditions are typically physical in nature - such as pressure, temperature, rate-of-fluid flow, and provide the physician or medical technician with critical information as to the status of a patient's condition.

One device that is widely used to monitor conditions is the blood pressure transducer. A blood pressure transducer senses the magnitude of a patient's blood pressure, and converts it into a representative electrical signal that is transmitted to the exterior of the patient. For most applications it is also required that the sensor is electrically energized.

Some means of signal and energy transmission is thus required, and most commonly extremely thin electrical cables, sometimes called microcables, are provided inside a guide wire, which itself is provided in the form of a tube, which often has an outer diameter in the order of 0.35 mm, and oftentimes is made of steel. In order to increase the bending strength of the tubular guide wire, a core wire is positioned inside the tube. The core wire also helps to improve "pushability" and "torquability" of the guide wire. The mentioned electrical cables are e.g. positioned in the space between the inner lumen wall and the core wire.

A potential problem with this kind of guide wire mounted sensors is the occurrence of so-called bending artefacts. A bending artefact is a change in the output signal from the sensor that is induced by a bending of the guide wire, rather than being induced by a change in the physical environment surrounding the sensor.

To achieve the desired resistance against bending artefacts, the sensor may be designed and mounted in different ways, the common feature being that it is a cantilevered mounting arrangement that provides the desired resistance against bending artefacts.

The term "cantilevered" means that one end of a structure is rigidly mounted, and the opposite end of the structure protrudes from the site of the mounting into a medium that is substantially less rigid than that at the mounting site.

Several different designs of sensor guide wires are known in the prior art, and examples of such sensor guide wires are disclosed in US 6167763 B1, which describes the cantilevered mounting of the sensor element, US RE39863 E1, which discloses the sensor element and US 6248083 B1, showing the complete sensor guide wire assembly, which all are assigned to the same assignee as in the present application.

US 2003/028128 A1 teaches a sensor guide wire according to present claim 1, but remains silent as to direct attachment of the outer surfaces of the core wire parts to the inner surface of the jacket. The cantilevered mounting of the sensor, however, requires extra machining or wire forming of the core wire at the site where the sensor chip is placed. The careful machining of the core wire into different diameters at different portions of the guide wire is a time-consuming process and may be a source of manufacturing mistakes, leading to loss of time and material in the manufacturing process.

Furthermore, in sensor guide wires used today, the sensor chip is often arranged in a short tube, also referred to as a jacket or a sleeve. The jacket is hollow and accommodates besides the sensor chip also a portion of a core wire and often at least one microcable. According to the prior art, the jacket is mainly used to protect the sensor chip.

Thus, there is a need for a sensor guide wire wherein the mounting of the sensor does not involve extra machining or wire forming, which thereby is easier and less expensive to manufacture.

### Summary of the invention

The above-mentioned objects are achieved by the present invention according to the independent claim.

Preferred embodiments are set forth in the dependent claims.

Thus, according to the present invention a sensor guide wire is provided which is less expensive to manufacture, quicker to assemble and in which the jacket serves a more structural role than in the prior art.

These objects of the present invention are achieved by a sensor guide wire having a divided core wire in the sensor region, and a jacket which connects a proximal region of the sensor guide wire with a tip region.

The sensor guide wire for intravascular measurements of physiological variables in a living body, in accordance with the present invention, has a proximal region, a distal sensor region and a tip region. The sensor guide wire comprises a core wire member, a sensor element, which has a sensor portion, for measuring the physiological variable and to generate a sensor signal in response to said variable, and a jacket, accommodating at least a part of said sensor element. The sensor portion is sensitive to one or many of the physiological variables pressure, temperature, and flow. The core wire member comprises two spatially separated parts, a first core wire part and a second core wire part, wherein a distal end of said first core wire part is attached to said jacket proximally said sensor portion and a proximal end of said second core wire part is attached to said jacket distally to said sensor portion.

### Short description of the appended drawings

Figure 1 shows the general design of a sensor guide wire according to the prior art.
Figure 2 shows the sensor guide wire according to the present invention, where the hollow tube is omitted for sake of simplicity.
Figure 3 shows the sensor guide wire according to the present invention.
Figure 4 shows a side view of the sensor guide wire according to the present invention.
Figure 5 shows the sensor guide wire according to the present invention, where the coil and the hollow tube have been omitted.
Figure 6 shows a side view of the first and second core wire parts attached to the jacket.
Figure 7 shows a cross section in the longitudinal direction of the sensor guide wire shown in figure 6.
Figure 8 shows a front view of the distal end of the sensor guide wire.
Figure 9 shows a cross-section A-A of the sensor region of the sensor guide wire shown in figure 6.
Figure 10 shows a side view of the sensor guide wire illustrating the first core wire part and the sensor element inside the jacket, according to one embodiment of the present invention.

Throughout the figures same reference signs designate the same, or essentially the same features.

### Detailed description of preferred embodiments of the invention

Throughout the application the word distal refers to the part located furthest away in respect of the operator, and the word proximal refers to the part located closest in respect of the operator.

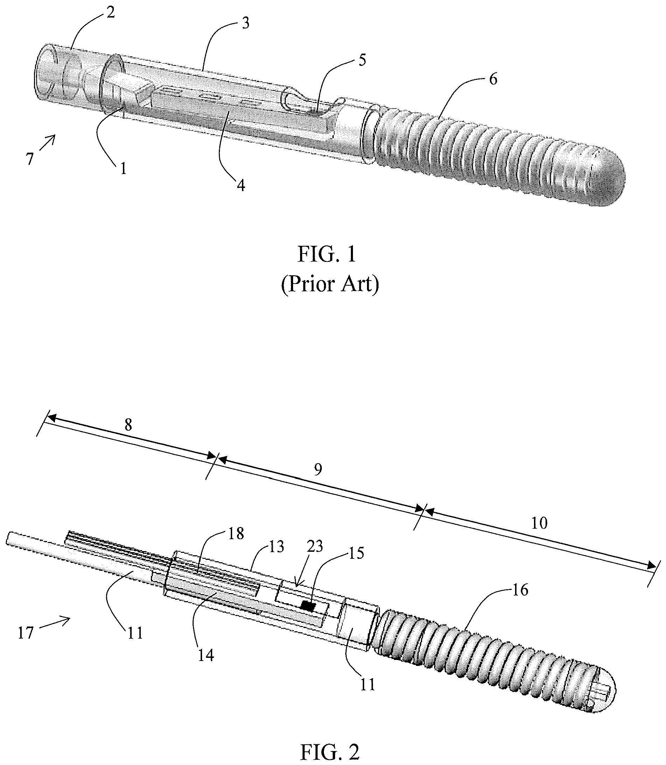

Figure 1 illustrates a sensor guide wire 7 according to the prior art. The sensor guide wire 7 comprises a core wire 1, a hollow tube 2, a hollow jacket 3, a sensor element 4 with a sensor portion 5, and a coil 6. The core wire 1 is at least partly disposed inside the hollow tube 2 and extends through the jacket 3 and into the coil 6. The sensor element 4, comprising the sensor portion 5, is mounted on the core wire 1 within the jacket 3, and is connected to an electronic unit (not shown in the figure) via one or several electrical leads (not shown in the figure).

In figure 2 is disclosed, a sensor guide wire 17 for intravascular measurements of physiological variables in a living body, having a proximal region 8, a distal sensor region 9 and a tip region 10, according to the present invention. The sensor guide wire 17 comprises a core wire member 11, a sensor element 14, which has a sensor portion 15, for measuring the physiological variable and to generate a sensor signal in response of said variable, and a jacket 13, accommodating at least a part of said sensor element 14. The sensor portion 15 is sensitive to one or many of the physiological variables, pressure, temperature, and flow. At least one signal transmitting microcable 18 is connected to said sensor element 14, and running along the sensor guide wire 17.

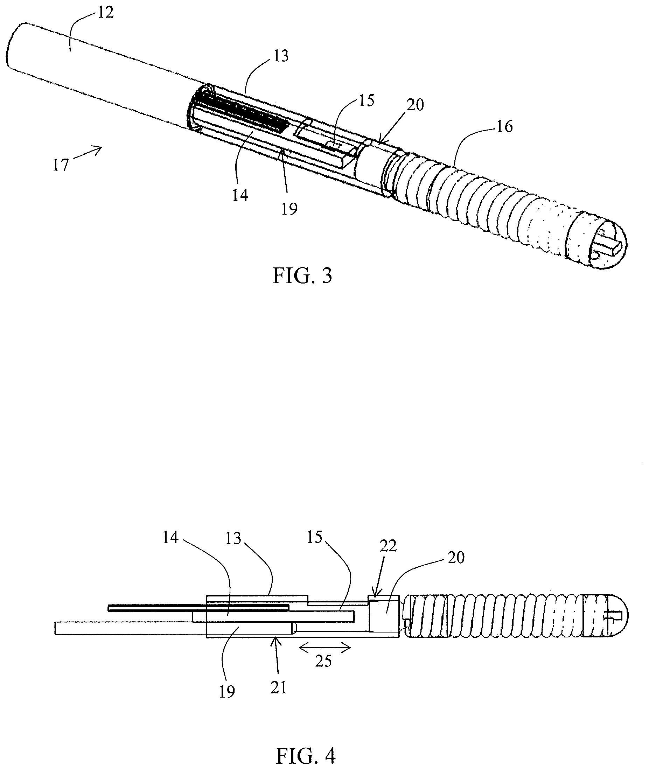

Furthermore, the sensor guide wire 17 comprises a coil 16 arranged in the tip region 10 and a hollow tube 12, as shown in figure 3, at least partly enclosing said signal transmitting microcable 18, in the proximal region 8. According to the present invention, the jacket 13 is provided with an aperture 23 in the sensor region 9, at the site, i.e the longitudinal section, where the sensor portion 15 is arranged, through which surrounding media may act on the sensor portion 15.

The core wire member 11 comprises two spatially separated parts, a first core wire part 19 and a second core wire part 20, as illustrated in figure 4. A distal end 21 of said first core wire part 19 is attached to said jacket 13 proximally said sensor portion 15 and a proximal end 22 of said second core wire part 20 is attached to said jacket 13 distally to said sensor portion 15. Thus, according to the embodiment shown in figure 4, the first core wire part 19 of the core wire is ended proximally the sensor portion 15 of the sensor element 14 and accordingly, there is no core wire at the site where the sensor portion 15 is arranged, i.e. in the longitudinal section of the sensor guide wire 17 where the sensor portion 15 is arranged.

As an obvious construction variation, the distal end 21 of the first core wire part 19 is attached to the jacket 13 proximally the entire sensor element 14.

A sensor guide wire 17 comprising a core wire member 11 having separate parts in the sensor region 9 is advantageous since no extra machining or wire shaping of the core wire member 11, at the site where the sensor element 14 is mounted, is needed.

The sensor is mounted in a cantilevering fashion such that an end comprising the sensor portion of the sensor does not contact any structure other than its mount. This prevents forces (bending artefacts) from being exerted on the sensor, which could otherwise interfere with measurements. Thus, the entire mounting structure provides a free space surrounding the distal part of the sensor element 14, this free space allowing air or blood or other e.g. pressure exerting media to enter the interior and to act on the sensor, which in its turn delivers a signal representative of the exerted pressure, the flow, and/or the temperature.

The length of the cantilevered portion 25 of the sensor element 14 (see figure 4) is between 0,1 to 2 mm, and preferably between 0,2 to 0,8 mm. The cantilevered portion 25 of the sensor element 14 is the part from where the first core wire part 19 of the core wire is ended to the distal end of the sensor element 14. As also shown in figure 4, the proximal end of the sensor element 14 in this embodiment protrudes proximally from the jacket 13. This embodiment facilitates the mounting of the hollow tube 12, since the hollow tube 12 (see figure 3) then may be threaded onto the proximal end of the sensor element 14, when being attached to the jacket 13.

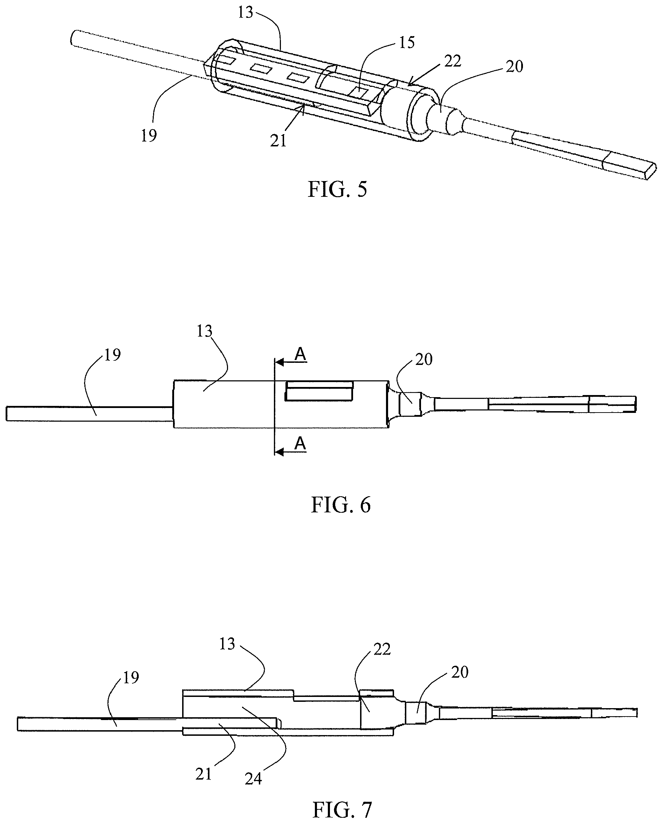

Figure 5 illustrates the sensor guide wire 17 according to the present invention, where the coil 16 and the hollow tube 12 have been omitted in order to be able to clearly illustrate the present invention. The distal end 21 of said first core wire part 19 is attached to said jacket 13 proximally said sensor portion 15, and the proximal end 22 of said second core wire part 20 is attached to said jacket 13 distally to said sensor portion 15. The jacket 13 thereby connects the proximal region 8 of the sensor guide wire 17 and the tip region 10.

As discussed above, by using the jacket 13 to connect the proximal region 8 and the tip region 10 has several advantages, for example, the sensor guide wire becomes less expensive to manufacture and quicker to assemble, and in addition it reduces the potential risk that bending artefacts of the core wire may influence the measurements.

In figure 6, a side view of the first and second core wire parts (19, 20) attached to the jacket 13, is disclosed.

In a preferred embodiment of the present invention, said distal and proximal ends (21, 22) of said first and second core wire parts (19, 20) are attached to said jacket 13 by means of welding.

According to another preferred embodiment of the present invention, said distal and proximal ends (21, 22) of said first and second core wire parts (19, 20) are attached to said jacket 13 by means of soldering.

According to yet another preferred embodiment of the present invention, said distal and proximal ends (21, 22) of said first and second core wire parts (19, 20) are attached to said jacket 13 by means of gluing. However, other suitable techniques may also be used in order attach the proximal and distal ends (21, 22) to the jacket 13. For example, the jacket 13 may be provided with a plurality of throughgoing holes, through which said distal and proximal ends (21, 22) of said first and second core wire parts (19, 20) and said jacket 13 is welded or soldered together.

Figure 7, which illustrates a cross section in the longitudinal direction of the sensor guide wire, shows that the distal end 21 of said first core wire part 19 may partly be inserted into the jacket 13 and fastened to the inner side 24 of the jacket 13. The cross-sectional dimension of the distal end 21 is in this embodiment less than the inner diameter of the jacket 13 in order to give space for the sensor element 14.

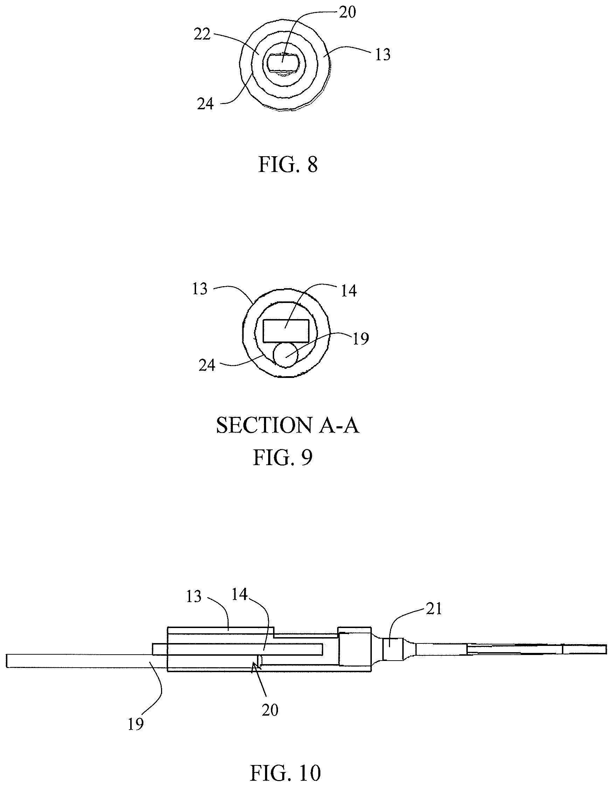

As illustrated in figures 8 and 10, the outer diameter of said proximal end 22 of said second core wire part 20 is adapted to the inner diameter of the jacket 13, so that the proximal end may partly be inserted into the jacket 13 and attached to the inner side 24 of the jacket 13 by welding, gluing or any other suitable technique. Alternatively, or in combination with welding, soldering, or gluing, said proximal end 22 may be attached to said jacket 13 by frictional engagement when inserted into said jacket 13.

As illustrated in figure 9, according to a preferred embodiment of the present invention, which shows a cross-section A-A of the sensor region 9 of the sensor guide wire 17 shown in figure 6, the jacket 13 is hollow and cylindrical and has a circular cross-section. The distal end 21 of said first core wire part 19 is attached to the inner side 24 of the hollow jacket 13 and the sensor element 14 is attached to the first core wire part 19, as may also be seen in figure 10.

The present invention is not limited to the above-described preferred embodiments. Various alternatives and modifications may be used. Therefore, the above embodiments should not be taken as limiting the scope of the invention, which is defined by the appending claims.

## Claims

1. Sensor guide wire (17) for intravascular measurements of physiological variables in a living body, which sensor guide wire (17) has a proximal region (8), a distal sensor region (9) and a tip region (10), the sensor guide wire (17) comprising :
- a core wire member (11);
- a sensor element (14), which has a sensor portion (15), configured to measure the physiological variable and to generate a sensor signal in response to said variable;
- a hollow jacket (13), which is cylindrical and has a circular cross-section, the jacket (13) surrounding at least a part of said sensor element (14);
wherein said core wire member (11) comprises two spatially separated parts, a first core wire part (19) and a second core wire part (20),
**characterized in that** an outer surface of a distal end (21) of said first core wire part (19) is directly attached to an inner surface of said jacket (13) proximally said sensor portion (15) and an outer surface of a proximal end (22) of said second core wire part (20) is directly attached to an inner surface of said jacket (13) distally to said sensor portion (15), and
that said first core wire part (19) extends at least partially along the proximal region (8), and
wherein said distal end (21) of said first core wire part (19) is ended proximally said sensor portion (15), and
that said second core wire part (20) extends at least partially along the tip region (10), and wherein said proximal end (22) of said second core wire part (20) is ended distally to said sensor portion (15), and
wherein at least the distal end (21) of the first core wire part (19) is offset from a longitudinal axis of the jacket (13), and the sensor element (14) is mounted in a cantilevered position on the offset distal portion of the first core wire part (19), such that the sensor element (14) has a cantilevered portion that overhangs a distal end of the first core wire part (19).

2. Sensor guide wire (17) according to claim 1, wherein no core wire member (11) is provided in the longitudinal section of the sensor guide wire (17) where said sensor portion (15) is arranged.

3. Sensor guide wire (17) according any of claims 1-2, wherein said sensor portion (15) is sensitive to one or many of the physiological variables pressure, temperature, and flow.

4. Sensor guide wire (17) according to any of claims 1-3, wherein said cantilevered portion (25) is between 0,1 to 2 mm, and preferably between 0,2 to 0,8 mm.

5. Sensor guide wire (17) according to any of claims 1-4, wherein said jacket (13) is provided with an aperture (23) in said sensor region (9), in the longitudinal section of the sensor guide wire (17) where said sensor portion (15) is arranged.

6. Sensor guide wire (17) according to any of claims 1-5, wherein said distal and proximal ends (21, 22) of said first and second core wire parts (19, 20) are attached to said jacket (13) by means of welding.

7. Sensor guide wire (17) according to any of claims 1-5, wherein said distal and proximal ends (21, 22) of said first and second core wire parts (19, 20) are attached to said jacket (13) by means of gluing.

8. Sensor guide wire (17) according to any of claims 1-7, comprising at least one signal transmitting microcable (18) connected to said sensor element (14), and running along the sensor guide wire (17).

## Patentansprüche

1. Sensor-Führungsdraht (17) für intravaskuläre Messungen von physiologischen Variablen in einem lebenden Körper, wobei der Sensor-Führungsdraht (17) eine proximale Region (8), eine distale Sensorregion (9) und eine Spitzenregion (10) aufweist, wobei der Sensor-Führungsdraht (17) umfasst:
- ein Kerndraht-Bauteil (11);
- ein Sensor-Element (14), welches einen Sensor-Abschnitt (15) aufweist, ausgelegt zum Messen der physiologischen Variablen und zum Erzeugen eines Sensorsignals in Reaktion auf die Variable;
- einen Hohlmantel (13), welcher zylindrisch ist und einen kreisförmigen Querschnitt aufweist, wobei der Mantel (13) mindestens einen Teil des Sensor-Elements (14) umgibt;
wobei das Kerndraht-Bauteil (11) zwei räumlich beabstandete Teile, ein erstes Kerndraht-Teil (19) und ein zweites Kerndraht-Teil (20), umfasst,
**dadurch gekennzeichnet, dass** eine äußere Oberfläche von einem distalen Ende (21) von dem ersten Kerndraht-Teil (19) direkt an einer inneren Oberfläche des Mantels (13) proximal zu dem Sensor-Abschnitt (15) befestigt ist und eine äußere Oberfläche von einem proximalen Ende (22) von dem zweitem Kerndraht-Teil (20) direkt an einer inneren Oberfläche des Mantels (13) distal zu dem Sensor-Abschnitt (15) befestigt ist, und
dass das erste Kerndraht-Teil (19) sich mindestens teilweise entlang der proximalen Region (8) erstreckt, und
wobei das distale Ende (21) von dem ersten Kerndraht-Teil (19) proximal zu dem Sensor-Abschnitt (15) endet, und
dass das zweite Kerndraht-Teil (20) sich mindestens teilweise entlang der Spitzenregion (10) erstreckt, und wobei das proximale Ende (22) von dem zweiten Kerndraht-Teil (20) distal zu dem Sensor-Abschnitt (15) endet, und
wobei mindestens das distale Ende (21) von dem ersten Kerndraht-Teil (19) von einer Längsachse des Mantels (13) verschoben ist, und das Sensor-Element (14) in einer ausladenden Position auf dem verschobenen distalen Abschnitt von dem ersten Kerndraht-Teil (19) befestigt ist, sodass das Sensor-Element (14) einen ausladenden Abschnitt aufweist, der über einem distalen Ende von dem ersten Kerndraht-Teil (19) hängt.

2. Sensor-Führungsdraht (17) nach Anspruch 1, wobei kein Kerndraht-Bauteil (11) in dem Längsabschnitt des Sensor-Führungsdrahts (17) bereitgestellt wird, in welchem der Sensor-Abschnitt (15) angeordnet ist.

3. Sensor-Führungsdraht (17) nach einem der Ansprüche 1 bis 2, wobei der Sensor-Abschnitt (15) auf eine oder viele der physiologischen Variablen Druck, Temperatur und Strömung empfindlich ist.

4. Sensor-Führungsdraht (17) nach einem der Ansprüche 1 bis 3, wobei der ausladende Abschnitt (25) zwischen 0,1 bis 2 mm und vorzugsweise zwischen 0,2 bis 0,8 mm ist.

5. Sensor-Führungsdraht (17) nach einem der Ansprüche 1 bis 4, wobei der Mantel (13) mit einer Öffnung (23) in der Sensor-Region (9) versehen ist, in dem Längsabschnitt von dem Sensor-Führungsdraht (17), in welchem der Sensor-Abschnitt (15) angeordnet ist.

6. Sensor-Führungsdraht (17) nach einem der Ansprüche 1 bis 5, wobei die distalen und proximalen Enden (21, 22) von den ersten und zweiten Kerndraht-Teilen (19, 20) an dem Mantel (13) durch Schweißen befestigt sind.

7. Sensor-Führungsdraht (17) nach einem der Ansprüche 1 bis 5, wobei die distalen und proximalen Enden (21, 22) von den ersten und zweiten Kerndraht-Teilen (19, 20) an dem Mantel (13) durch Kleben befestigt sind.

8. Sensor-Führungsdraht (17) nach einem der Ansprüche 1 bis 7, der mindestens ein Signal-Übertragungs-Mikrokabel (18) umfasst, das mit dem Sensor-Element (14) verbunden ist, und entlang des Sensor-Führungsdrahts (17) verläuft.

## Revendications

1. Fil de guidage de capteur (17) pour des mesures intravasculaires de variables physiologiques dans un corps vivant, dont le fil de guidage de capteur (17) a une région proximale (8), une région de capteur distale (9) et une région de pointe (10), le fil de guidage de capteur (17) comprenant :
- un membre de fil de noyau (11) ;
- un élément de capteur (14), qui a une portion de capteur (15), configuré pour mesurer la variable physiologique et pour générer un signal de capteur en réponse à ladite variable ;
- une enveloppe creuse (13), qui est cylindrique et a une section transversale circulaire, l'enveloppe (13) entourant au moins une partie dudit élément de capteur (14) ;
dans lequel ledit membre de fil de noyau (11) comprend deux parties spatialement séparées, une première partie de fil de noyau (19) et une deuxième partie de fil de noyau (20),
**caractérisé en ce qu'**une surface extérieure d'une extrémité distale (21) de ladite première partie de fil de noyau (19) est directement attachée à une surface intérieure de ladite enveloppe (13) à proximité de ladite portion de capteur (15) et une surface extérieure d'une extrémité proximale (22) de ladite deuxième partie de fil de noyau (20) est directement attachée à une surface intérieure de ladite enveloppe (13) à distance de ladite portion de capteur (15), et
**en ce que** ladite première partie de fil de noyau (19) s'étend au moins partiellement le long de la région proximale (8), et
dans lequel ladite extrémité distale (21) de ladite première partie de fil de noyau (19) se termine à proximité de ladite portion de capteur (15), et
**en ce que** ladite deuxième partie de fil de noyau (20) s'étend au moins partiellement le long de la région de pointe (10), et dans lequel ladite extrémité proximale (22) de ladite deuxième partie de fil de noyau (20) se termine à distance de ladite portion de capteur (15), et
dans lequel au moins l'extrémité distale (21) de la première partie de fil de noyau (19) est décalée par rapport à un axe longitudinal de l'enveloppe (13), et l'élément de capteur (14) est monté dans une position en porte-à-faux de la portion distale décalée de la première partie de fil de noyau (19), de sorte que l'élément de capteur (14) a une portion en porte-à-faux qui dépasse une extrémité distale de la première partie de fil de noyau (19).

2. Fil de guidage de capteur (17) selon la revendication 1, dans lequel aucun membre de fil de noyau (11) n'est fourni dans la section longitudinale du fil de guidage de capteur (17) où ladite portion de capteur (15) est arrangée.

3. Fil de guidage de capteur (17) selon l'une quelconque des revendications 1 à 2, dans lequel ladite portion de capteur (15) est sensible à une ou plusieurs des variables physiologiques pression, température, et circulation.

4. Fil de guidage de capteur (17) selon l'une quelconque des revendications 1 à 3, dans lequel ladite portion en porte-à-faux (25) est entre 0,1 à 2 mm, et préférablement entre 0,2 à 0,8 mm.

5. Fil de guidage de capteur (17) selon l'une quelconque des revendications 1 à 4, dans lequel ladite enveloppe (13) est fournie avec une ouverture (23) dans ladite région de capteur (9), dans la section longitudinale du fil de guidage de capteur (17) où ladite portion de capteur (15) est arrangée.

6. Fil de guidage de capteur (17) selon l'une quelconque des revendications 1 à 5, dans lequel lesdites extrémités distale et proximale (21, 22) desdites première et deuxième parties de fil de noyau (19, 20) sont attachées à ladite enveloppe (13) au moyen d'une soudure.

7. Fil de guidage de capteur (17) selon l'une quelconque des revendications 1 à 5, dans lequel lesdites extrémités distale et proximale (21, 22) desdites première et deuxième parties de fil de noyau (19, 20) sont attachées à ladite enveloppe (13) au moyen d'un collage.

8. Fil de guidage de capteur (17) selon l'une quelconque des revendications 1 à 7, comprenant au moins un micro-câble transmetteur de signaux (18) connecté audit élément de capteur (14), et s'étendant le long du fil de guidage de capteur (17).
